# EUROPEAN PATENT APPLICATION

(11) **EP 3 434 250 A1**
(43) Date of publication of application: **30.01.2019**
(21) Application number: 17382492.1
(22) Date of filing: 25.07.2017
(51) Int. Cl.: A61H 23/00, A61M 21/00

(54) **SYSTEM AND METHOD FOR DISTRIBUTED SENSORIAL STIMULATION**

(71) Applicant: Sensorial Processing Technology Barcelona, SL, 08010 Barcelona (ES)
(72) Inventor: ORLACH ENRIQUEZ, Victor, 08015 Barcelona (ES)
(74) Representative: Juncosa Miró, Jaime

(57) **Abstract**

The present invention relates to a system and method for distributed sensory stimulation, the system comprising a control unit (10) for receiving an electrical audio signal and for distributing said signal into a plurality of sensory signals, and a plurality of actuators (20L, 20R, 21L, 21R, 22L, 22R, 23L, 23R, 24L, 24R) connected to said control unit (10) and configured to receive the sensory signals, acting by providing sensory stimulation in specific regions of a user's body, wherein the regions of the user's body comprise three regions involved in human perception ranging from the ears, the lower and upper limbs and the torso and pelvis; the actuators comprise eight vibration actuators and two auditory actuators producing said sensory stimulation in the three regions of the user; and the control unit (10) performs delivery considering the vibrational capturing capacity of each of said three regions depending on the volume and structural density thereof.

## Description

### Field of the Art

The present invention generally relates to systems and methods for sensory stimulation. Particularly, the invention relates to a system and method for distributed sensory stimulation particularly adapted to each region involved in human perception.

Sensorialism must be understood as the technical and human capacity which comprises the (analog and/or digital) transmission and processing of any perception that can be perceived by a human being in real time.

"Sensory signal" will be understood herein as a filtered or conditioned electrical signal capable of interacting with an actuator for transferring a vibrating sensation, tactile sensation or auditory sensation to a region of the user's body.

### Background of the Invention

Patent application US-A1-2016317383 discloses a garment including sensor devices for auditory and tactile stimulation. The actuators allow a precise user stimulation determined by their ability to conform to the scientific methodology of repeatability, reproducibility and reliability.

Patent application US-A1-20110251535 discloses an apparatus and a method for introducing multisensory integration. The apparatus includes an ergonomically contoured seating device, at least one vibrating acoustic device, at least one plate for dispersing vibration throughout the entire seating device, a rotatable mechanism for rotating the seating device.

Patent application US-A1-2014010387 discloses an apparatus capable of transmitting sound and vibrations to a user. The apparatus may also generate an electromagnetic field sufficient to stimulate the user's energy system. The apparatus includes a support structure configured to support at least a part of the user's body; a transducer coupled to the frame of the support structure in a manner which allows the transducer to move in relation to the frame; and an audio signal source for transmitting audio signals to the transducer. The transducer is capable of receiving and responding to audio signals having a wide range of audio frequencies when the apparatus is in use.

However, none of the prior art documents considers a distributed sensory stimulation, making a distinction for each region of a user, depending on the particular characteristics of each region. Likewise, the prior art documents do not allow simultaneous stimulation of different regions of a user including the lower and upper limbs, the torso and pelvis and the ears.

There is therefore a need for new systems and methods for an efficient distributed sensory stimulation according to the frequencies comprised within the perception limits of each region of the body.

### Brief Description of the Invention

In a first aspect, the present invention provides a system for distributed sensory stimulation which comprises, like the solutions of the state of the art, a control unit and a plurality of actuators. The mentioned control unit is configured to receive at least one analog electrical audio signal and to distribute said received audio signal into a plurality of sensory signals, and the plurality of actuators, which are operatively connected to the control unit, are configured to receive the mentioned plurality of sensory signals, acting by providing sensory stimulation in specific regions of a user's body.

Unlike the known solutions, in the proposed system the regions of the user's body which will interact with the actuators comprise several distinct regions, including three regions involved in human perception ranging from the ears, the lower and upper limbs and the torso and pelvis. Likewise, the plurality of actuators comprises at least eight vibration actuators and two auditory actuators, such as headphones or directional loudspeakers, producing said distinct sensory stimulation in the three regions of the user. Preferably, four vibration actuators are included for a first region of said three regions which is demarcated by the lower and upper limbs (i.e., an actuator for the right arm, an actuator for the left arm, an actuator for the right leg and an actuator for the left leg), four vibration actuators are included for a second region demarcated by the torso and pelvis (i.e., two actuators for the right-hand side of the body between the torso and pelvis and two actuators for the left-hand side of the body), and two auditory actuators are included for a third region demarcated by the ears. It must be indicated that the proposed system can include a larger number of vibration actuators, i.e., more than one actuator per limb for the first region or more than two actuators per side for the second region, said two or more actuators operating in a synchronized manner or with a very slight difference in each area.

Furthermore, in the proposed system the control unit is configured to deliver the received audio signal to said vibration and auditory actuators taking into consideration the vibrational capturing capacity of each of said three regions depending on the volume and structural density thereof, wherein the four sensory signals for stimulating the first region are comprised in the range of [0-100] Hz, the four sensory signals for stimulating the second region are comprised in the range of [100-400] Hz, and the two sensory signals for stimulating the third region are comprised in the range of [0-20] kHz.

In a preferred embodiment, the eight vibration actuators are arranged in a common support structure which accommodates the user, for example an armchair or the like.

In one embodiment, the received audio signal is synchronized with a visual and auditory signal, for example a video signal, depicted on a display, for example of a television, a computer, a mobile telephone, a tablet, etc., accessible by the user.

Preferably, the control unit is implemented by means of a demultiplexer circuit formed by eight filtering units, one for each of the eight vibration actuators.

In one embodiment, the system further incorporates three adjustment devices, such as potentiometers, one for each of the three regions of the user's body to be stimulated, configured to perform adjustment by amplifying or attenuating the sensory stimulation produced by each vibration and auditory actuator in said regions.

According to the invention, the audio signal can comprise an analog signal received by means of an audio connector, for example a jack connector, connected to the control unit. Alternatively, the audio signal can comprise an analog signal received wirelessly through the control unit, preferably by means of a Bluetooth communication system.

In a second aspect, the embodiments of the present invention provide a method for distributed sensory stimulation which comprises a control unit receiving an electrical audio signal; said control unit distributing the received audio signal into a plurality of sensory signals; and a plurality of actuators operatively connected to the control unit receiving the plurality of sensory signals, acting by providing sensory stimulation in specific regions of a user's body.

According to the proposed method, the mentioned regions of the user's body comprise several distinct regions including three regions involved in human perception ranging from the ears, the lower and upper limbs and the torso and pelvis, and the plurality of actuators comprise eight synchronized vibration actuators and two auditory actuators also with synchronized outputs (right/left) to perform sensory stimulation in the three regions of the user in a synchronized manner. Likewise, the delivery of the received audio signal is performed taking into consideration the vibrational capturing capacity of each of said three regions depending on the volume and structural density thereof.

The tests conducted by the inventors of the present invention have demonstrated that human perception variables consisting of the volume and structural density of the regions to be stimulated are the two most important factors to be considered when performing stimulation. The first variable is the volumetric surface of the region of the human body and the second variable is the internal material adjacent to said region, made up of tissues, muscles and bones and the cycles of movement thereof which are reflected in terms of different measurements and functionality. To that end, the four signals for stimulating the first region are comprised in the range of [0-100] Hz, the four signals for stimulating the second region are comprised in the range of [100-400] Hz, and the two signals for stimulating the third region are comprised in the range of [0-20] kHz.

In one embodiment, the proposed method comprises showing a visual and auditory signal on a display at the same time as receiving the audio signal.

In one embodiment, the method also comprises amplifying or attenuating the sensory stimulation produced by each vibration or auditory actuator in the mentioned three regions (over a period of application) by means of three adjustment devices, one for each region of the user's body.

Therefore, the present invention provides a technological device which demultiplexes (distributes) an electrical audio signal for emitting same to actuators according to human vibration acquisitions of each of the regions in contact with the actuators. The user can also control the output provided to the actuators through their CPU or potentiometers.

The invention allows reproducing the audio content received using any computer, mobile telephone, music player, etc.

Therefore, the present invention provides a new sensory technology which synchronizes and processes in real time audio, video and vibrations synchronized with one another, and generates distributed stimulation in different regions of a user's body from one and the same audio signal, with specific patterns identified and obtained during processing, opening up a new technological development as it allows stimulating the user's whole body in a distinctive manner due to the action of the different actuators connected to the control unit.

### Brief Description of the Drawings

The foregoing and other features and advantages will be better understood based on the following detailed description of several merely illustrative and non-limiting embodiments in reference to the attached drawings, in which:
Figure 1 shows a schematic view of a system for distributed sensory stimulation according to an embodiment of the present invention.
Figure 2 is a block diagram showing a method for distributed sensory stimulation according to an embodiment of the present invention.

### Detailed Description of Exemplary Embodiments

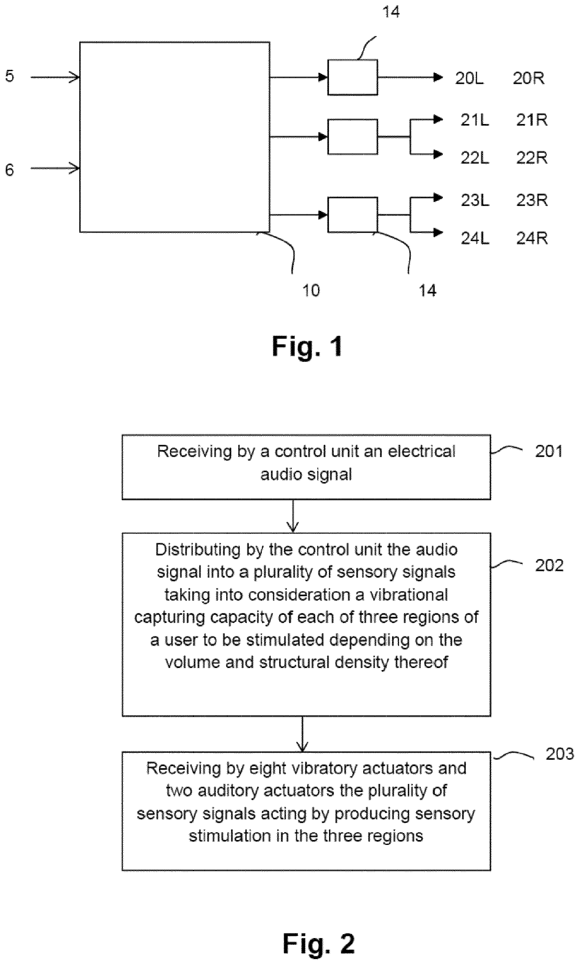

Figure 1 shows a preferred embodiment of the proposed system (or device). According to this preferred embodiment, the system includes a control unit 10 which receives, either by means of an audio connector such as a jack connector or wirelessly, for example, by means of Bluetooth or a similar wireless technology, an audio signal 6 and distributes it into different sensory signals for application thereof through actuators 20L, 20R, 21L, 21R, 22L, 22R, 23L, 23R, 24L, 24R to three distinct regions of the user's body (not illustrated for the sake of simplicity in the drawing), including the ears, the lower and upper limbs and the torso and pelvis. The control unit 10 includes a feed connector for feeding same by means of an electrical signal 5, such as a Vdc signal.

According to this preferred embodiment, the mentioned actuators 20L, 20R, 21L, 21R, 22L, 22R, 23L, 23R, 24L, 24R comprise eight vibration actuators 21L, 21R, 22L, 22R, 23L, 23R, 24L, 24R and two auditory actuators 20L, 20R, for example headphones or directional loudspeakers. In other words, four vibration actuators 21L, 21R, 22L, 22R are included for the region demarcated by the lower and upper limbs (or first region), four vibration actuators 23L, 23R, 24L, 24R are included for the region demarcated by the torso and pelvis (or second region) and two auditory actuators 20L, 20R are included for the air region demarcated by the ears (or third region). In other embodiments not illustrated, the system includes more than one vibration actuator for each of the lower and upper limbs. Likewise, the system can also include a different configuration of actuators for the second region, for example including more than two actuators for the (left- and right-hand) side of said second region of the user's body.

The control unit 10 of the embodiment of Figure 1 comprises a demultiplexer circuit formed in this case by eight filtering units, one for each of the eight vibration actuators 21L, 21R, 22L, 22R, 23L, 23R, 24L, 24R.

According to this preferred embodiment, the system also (optionally) incorporates three adjustment devices 14, for example potentiometers, allowing the user to adjust the sensory stimulation produced by each actuator 20L, 20R, 21L, 21R, 22L, 22R, 23L, 23R, 24L, 24R in each of the three regions, for example changing the volume or vibration.

The eight vibration actuators 21L, 21R, 22L, 22R, 23L, 23R, 24L, 24R are preferably arranged in a common support structure which can accommodate the user, such as an armchair or the like.

In one embodiment, the received audio signal 6 is synchronized with a visual and auditory signal depicted on a display, for example of a television or mobile telephone, advantageously accessible by the user. In other words, the audio signal acting as an input signal of the control unit 10, for example, the audio component of a video from an Internet portal which allows uploading and playing videos, is synchronized with the component of said video.

Figure 2 illustrates an embodiment of the proposed method. According to this particular embodiment, the method comprises step 201 consisting of the control unit 10 receiving an electrical audio signal, then step 202 consisting of the control unit 10 distributing the received audio signal 6 into a plurality of sensory signals taking into consideration the specific vibrational capturing capacity for each of the three regions of the user to be stimulated depending on the volume and structural density thereof, and finally step 203 consisting of the eight vibration actuators and the two auditory actuators receiving the plurality of sensory signals, acting by providing sensory stimulation in each of the three regions of the user's body.

According to the invention, the delivery of the sensory signals is performed taking into account the particular stimulation frequencies which can be tolerated by each region of the body of the user. In other words, the four signals for stimulating the first region are comprised in the range of [0-100] Hz, the four signals for stimulating the second region are comprised in the range of [100-400] Hz, and the two signals for stimulating the third region are comprised in the range of [0-20] kHz.

The scope of the present invention is defined in the attached claims.

## Claims

1. A system for distributed sensory stimulation, comprises:
a control unit (10) configured to receive at least one electrical audio signal (6) and to distribute said received audio signal (6) into a plurality of sensory signals; and
a plurality of actuators (20L, 20R, 21L, 21R, 22L, 22R, 23L, 23R, 24L, 24R) operatively connected to said control unit (10) and configured to receive said plurality of sensory signals, acting by providing sensory stimulation in specific regions of a user's body,
**characterized in that**:
said specific regions of the user's body comprise several distinct regions including three regions involved in human perception ranging from the ears, the lower and upper limbs and the torso and pelvis;
said plurality of actuators (20L, 20R, 21L, 21R, 22L, 22R, 23L, 23R, 24L, 24R) comprises at least eight vibration actuators (21L, 21R, 22L, 22R, 23L, 23R, 24L, 24R) and two auditory actuators (20L, 20R) producing said sensory stimulation in the three regions of the user, such that four vibration actuators (21L, 21R, 22L, 22R) are included for a first region of said three regions which is demarcated by the lower and upper limbs, four vibration actuators (23L, 23R, 24L, 24R) are included for a second region of said three regions which is demarcated by the torso and pelvis and two auditory actuators (20L, 20R) are included for a third region of said three regions which is demarcated by the ears; and **in that**
said control unit (10) is configured to deliver the received audio signal (6) to said vibration and auditory actuators (20L, 20R, 21L, 21R, 22L, 22R, 23L, 23R, 24L, 24R) taking into consideration the vibrational capturing capacity of each of said three regions depending on the volume and structural density thereof, wherein:
the four sensory signals for stimulating the first region are comprised in the range of [0-100] Hz,
the four sensory signals for stimulating the second region are comprised in the range of [100-400] Hz, and
the two sensory signals for stimulating the third region are comprised in the range of [0-20] kHz.

2. The system according to claim 1, wherein the received audio signal (6) is synchronized with a visual and auditory signal depicted on a display.

3. The system according to claim 1, wherein said control unit (10) comprises a demultiplexer circuit formed by at least eight filtering units for each of the eight vibration actuators.

4. The system according to claim 1, further comprising at least three adjustment devices (14), one for each of the three regions of the user's body, configured to amplify or attenuate for a specific selectable time period the sensory stimulation produced by each vibration and auditory actuator (20L, 20R, 21L, 21R, 22L, 22R, 23L, 23R, 24L, 24R) in said regions.

5. The system according to claim 1, wherein the audio signal (6) comprises an analog signal received by means of an audio connector connected to the control unit (10).

6. The system according to claim 1, wherein the audio signal (6) comprises an analog signal received wirelessly through the control unit (10).

7. The system according to claim 6, wherein said analog signal is received by means of a Bluetooth communication system.

8. The system according to claim 1, wherein the eight vibration actuators (21L, 21R, 22L, 22R, 23L, 23R, 24L, 24R) are arranged in a support structure which accommodates the user.

9. The system according to claim 8, wherein said support structure comprises at least one armchair.

10. A method for distributed sensory stimulation, comprising:
a) receiving by a control unit (10) at least one electrical audio signal (6);
b) distributing by said control unit (10) said received audio signal into a plurality of sensory signals; and
c) receiving, by a plurality of actuators (20L, 20R, 21L, 21R, 22L, 22R, 23L, 23R, 24L, 24R) operatively connected to the control unit (10), said plurality of sensory signals, acting by providing sensory stimulation in specific regions of a user's body,
**characterized in that**:
said specific regions of the user's body comprise several distinct regions including three regions involved in human perception ranging from the ears, the lower and upper limbs and the torso and pelvis;
said plurality of actuators (20L, 20R, 21L, 21R, 22L, 22R, 23L, 23R, 24L, 24R) comprises at least eight vibration actuators (21L, 21R, 22L, 22R, 23L, 23R, 24L, 24R) and two auditory actuators (20L, 20R) producing said sensory stimulation in the three regions of the user, such that four vibration actuators (21L, 21R, 22L, 22R) act in a first region of said three regions which is demarcated by the lower and upper limbs, four vibration actuators (23L, 23R, 24L, 24R) act in a second region of said three regions which is demarcated by the torso and pelvis and two auditory actuators (20L, 20R) act in a third region of said three regions which is demarcated by the ears; and
distribution of the received audio signal is performed taking into consideration the vibrational capturing capacity of each of said three regions depending on the volume and structural density thereof, wherein:
the four signals for stimulating the first region are comprised in the range of [0-100] Hz,
the four signals for stimulating the second region are comprised in the range of [100-400] Hz, and
the two signals for stimulating the third region are comprised in the range of [0-20] kHz.

11. The method according to claim 10, comprising showing a visual and auditory signal on a display at the same time as receiving the audio signal.

12. The method according to claim 11, further comprising amplifying the sensory stimulation produced by each vibration or auditory actuator (20L, 20R, 21L, 21R, 22L, 22R, 23L, 23R, 24L, 24R) in said three regions by means of three adjustment devices (14), one for each region of the user's body.
